(19) Europäisches Patentamt
European Patent Office
Office européen des brevets

(11) Publication number: **0 426 075 A1**

(12) **EUROPEAN PATENT APPLICATION**

(21) Application number: **90120713.4**

(51) Int. Cl.[5]: **C07D 207/08**, A61K 31/40

(22) Date of filing: **29.10.90**

(30) Priority: **30.10.89 US 428577**

(43) Date of publication of application:
**08.05.91 Bulletin 91/19**

(84) Designated Contracting States:
**AT BE CH DE DK ES FR GB GR IT LI LU NL SE**

(71) Applicant: **SYNTEX (U.S.A.) INC.**
**3401 Hillview Avenue**
**Palo Alto California 94304(US)**

(72) Inventor: **Fisher, Lawrence E.**
**1036 Marilyn Drive**
**Mountain View, California 94040(US)**
Inventor: **Caroon, Joan M.**
**1936 Silverwood**
**Mountain View, California 94043(US)**
Inventor: **Muchowski, Joseph M.**
**1720 Banff Drive**
**Sunnyvale, California 94087(US)**
Inventor: **Rosenkranz, Roberto P.**
**7 Brittany Meadows**
**Atherton, California 94027(US)**
Inventor: **McClelland, Deborah L.**
**4119 Park Boulevard**
**Palo Alto, California 94306(US)**

(74) Representative: **Barz, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

(54) **Benzylpyrrolidine derivatives as dopamine agonists.**

(57) Dopamine agonist compounds disclosed are useful in treating hypertension and congestive heart failure in mammals. The compounds have the following general formula (I)

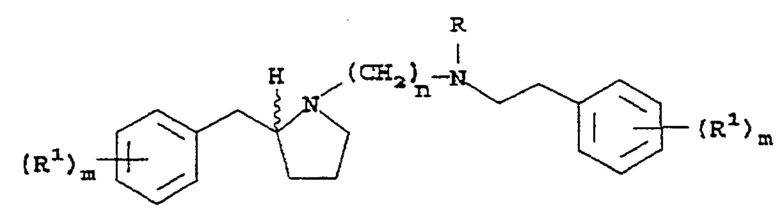

(I)

wherein:
R is hydrogen or lower alkyl;
each $R^1$ is independently hydrogen,

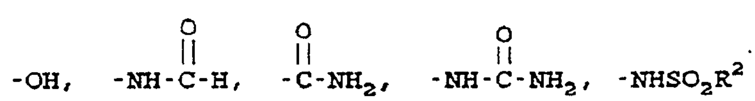

EP 0 426 075 A1

or halo;
$R^2$ is lower alkyl or optionally substituted phenyl; m is an integer of 0, 1, 2, 3, 4, 5, or 6; and n is an integer of from 1 to 10; or a pharmaceutically acceptable salt, (R) or (*S*) stereoisomers, or racemic or non-racemic mixtures thereof.

# BENZYLPYRROLIDINE DERIVATIVES AS DOPAMINE AGONISTS

The invention relates generally to dopamine agonists and more particularly to benzylpyrrolidine derivatives which act as dopamine agonists and as such are useful in the treatment of hypertension, congestive heart failure, acute and chronic renal failure, angina and hyperprolactenemia in mammals. The invention also relates to methods of making such compounds, pharmaceutical dosage forms comprising such compounds and to methods of treatment involving the administration of such compounds to a mammal.

Drugs having the pharmacological effect of dopamine are referred to as dopaminergic agonists in that dopamine is the only marketed sympathomimetic with significant dopaminergic actions in the periphery; some sympathomimetics appear to act on dopamine receptors in the central nervous system. In the periphery, dopamine receptors are prominent in the splanchnic and renal vascular beds, where they mediate vasodilatation. Dilation in these beds is important in the treatment of shock and acute heart failure, since these beds are often critically constricted in these conditions. Dopamine is used in the management of these disorders. It may also be used to induce diuresis, probably consequent to renal vasodilatation, at least in part.

DOPAMINE HYDROCHLORIDE

4-(2-aminoethyl)-1,2-Benzenediol hydrochloride;

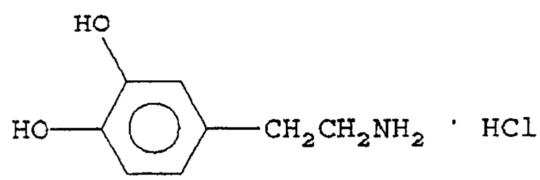

also known as 3,4-Dihydroxyphenethylamine hydrochloride

Dopamine is the immediate precursor of norepinephrine. This catecholamine has important functions as a chemical mediator in some parts of the central nervous system. In addition it has been introduced as a therapeutic agent under the trade name Intropin.

Dopamine acts on beta receptors in the heart, causing increased contractility and heart rate. In large enough doses it acts on alpha receptors in blood vessels, causing vasoconstriction. Dopamine exerts some unusual vasodilator effects on the renal, mesenteric, coronary, and intracerebral vessels, which suggest the existence of specific dopaminergic receptors. These are inhibited by haloperidol and the phenothiazines. These dopamine vascular receptors may be similar to the dopaminergic receptors in the basal ganglia.

The hemodynamic effects of dopamine depend on the dose with some individual variations. Intravenous infusion of 1 to 10 µg/kg/min lead to increased cardiac contractility, cardiac output, and renal blood flow. Heart rate and mean blood pressure do not change significantly. With higher infusion rates arterial pressure rises and heart rate decreases.

Dopamine infusions are used in some cases of shock and in chronic refractory congestive failure. Ventricular arrhythmia is the most serious adverse effect. Nausea, vomiting, and hypotension may also occur. The action of the drug is dissipated in a few minutes.

U.S. Patent 4,613,606 issued September 23, 1986 discloses a number of tetrahydroisoquinoline derivatives which are indicated as being calcium channel blockers and as such useful for the treatment of cardiovascular disorders including angina, hypertension and congestive heart failure.

European Patent Application No. 294,973 published December 14, 1988 discloses a number of dopamine-beta-hydroxylase inhibitors (DBH inhibitors). Dopamine is hydroxylated to norepinephrine by (DBH) in the presence of oxygen and ascorbic acid. There are a number of known DBH inhibitors discussed in 294,973 which are believed to be effective in treating hypertension. Methods of synthesizing the novel compounds are also disclosed.

European Patent Application No. 235,463 published September 9, 1987 discloses a series of N-substituted arylalkyl and arylalkylene pyrrolidines, piperidines and homopiperidines useful as cardiovascular, antihistaminic and antisecretory agents.

European Patent Application No. 71,399 discloses a series of 2,5-disubstituted pyrrolidines useful as cardiovascular agents and bronchodilators with prolonged activity.

A primary object of the present invention is to disclose and provide novel compounds represented by the following general structural formula (I):

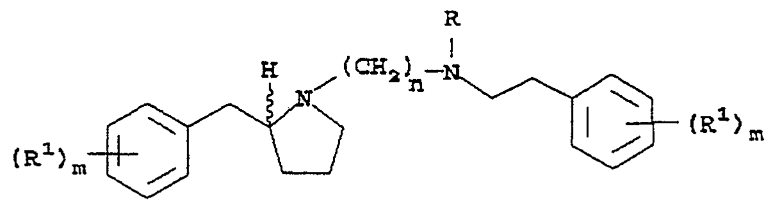

(I)

wherein:
R is hydrogen or lower alkyl;
each $R^1$ is independently hydrogen,

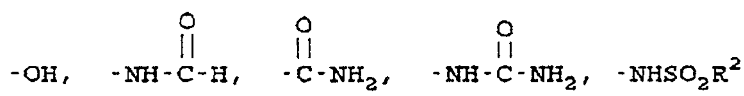

or halo;
$R^2$ is lower alkyl or optionally substituted phenyl; m is an integer of 0, 1, 2, 3, 4, 5, or 6; and n is an integer of from 1 to 10; or pharmaceutically acceptable salts, (*R*) or (*S*) stereoisomers or racemic or non-racemic mixtures thereof.

In formula I each $R_1$ is preferably hydrogen or -OH with most preferably at least three -OH groups being present on the molecule (m = 3), R is preferably a propyl moiety, and n is preferably 5 or 6.

The dopamine agonist is preferably in the form of a pharmaceutically acceptable salt of a compound of formula (I) which salt is preferably included in a pharmaceutical dosage form by combining the salt with a suitable carrier system - most preferably a carrier system suitable for oral delivery. A mammal can be treated by administering the compound of formula (I) in its dosage form and thereby obtaining the pharmacological effect of the drug on the mammal.

Another object is to provide racemic and non-racemic mixtures of the compounds of formula (I) as well as the (*S*) and (*R*) stereoisomers.

Another object of the invention is to disclose and provide novel methods of preparing the compounds of formula (I) or their salts as well as the novel intermediates produced by such methods.

For example, a process for the preparation of compounds of formula I:

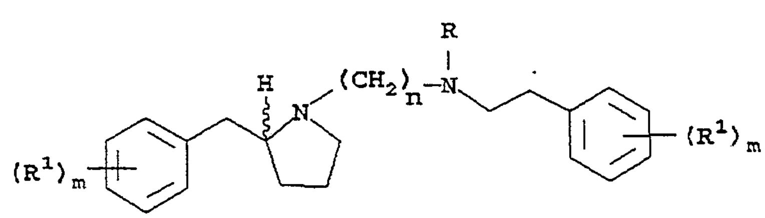

(I)

wherein:
R is hydrogen or lower alkyl;

each $R^1$ is independently hydrogen,

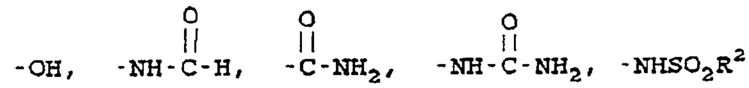

or halo;

$R^2$ is lower alkyl or optionally substituted phenyl; m is an integer of 0, 1, 2, 3, 4, 5, or 6; n is an integer of from 1 to 10; or pharmaceutically acceptable salts, (R) or (S) stereoisomers or racemic or non-racemic mixtures thereof;

which comprises

a) reacting a compound of formula 14

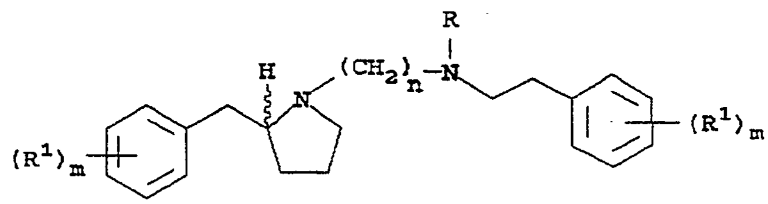

(14)

wherein $R^1$ is OR, R, m, and n are as defined above with a deprotecting agent; or

b) converting a compound of formula I to a pharmaceutically acceptable salt of formula I; or

c) converting a pharmaceutically acceptable salt of formula I to a free compound of formula I; or

d) converting a pharmaceutically acceptable salt of formula I to another pharmaceutically accpetable salt of formula I; or

e) resolving a compound of formula I into its (R) and (S) stereoisomers thereof.

For example, a compound of the formula

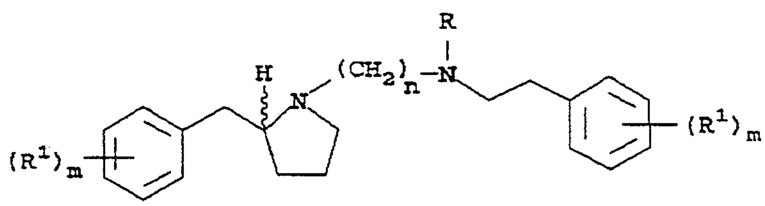

(I)

wherein:

R is hydrogen or lower alkyl;

each $R^1$ is a protected hydroxy group; m is an integer of 0, 1, 2, 3, 4, 5, or 6; and n is an integer of from 1 to 10; or a pharmaceutically acceptable salt, (R) or (*S*) stereoisomers, or racemic or non-racemic mixtures thereof.

For example, a process for the preparation of a compound of the formula

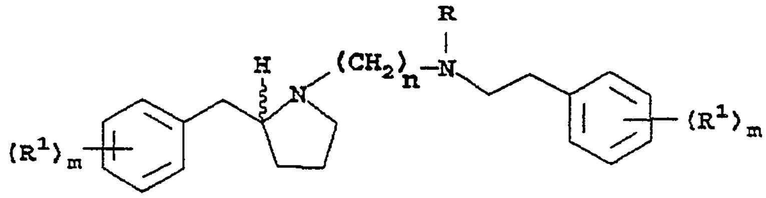

(I)

wherein:

R is hydrogen or lower alkyl;

each $R^1$ is a protected hydroxy group; m is an integer of 0, 1, 2, 3, 4, 5, or 6; and n is an integer of from 1 to 10; or a pharmaceutically acceptable salt, (R) or (*S*) stereoisomers, or racemic or non-racemic mixtures thereof

which comprises reacting a compound of the formula

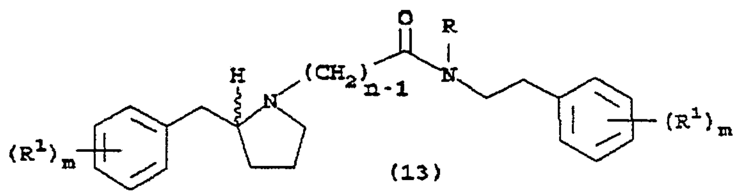

(13)

wherein $R^1$, m, and n are as described above, with a reducing agent.

Yet another object is to disclose and provide pharmaceutical compositions and dosage forms containing compounds of formula (I) or their salts with pharmaceutically acceptable non-toxic carriers.

Still another object of the invention is to disclose and provide methods of treating hypertension, congestive heart failure, acute and chronic renal failure, angina and hyperprolactenemia in mammals by administering to the mammals the dosage forms of the invention.

A feature of the present invention is that compounds of formula (I) and the pharmaceutically acceptable salts thereof are orally active.

Another feature of the present invention is that the compounds of formula (I) can be produced via an efficient process and that the processes disclosed allow for the production of racemic mixtures or optically pure stereoisomers.

An advantage of the present invention is that the compounds of formula (I) are very effective dopamine agonists.

An important advantage of the compounds of this invention is that they can be administered orally to treat hypertension, congestive heart failure, acute and chronic renal failure, angina and hyperprolactenemia.

These and other objects, advantages and features of the present invention will become apparent to those persons skilled in the art upon reading the details of the various compounds, and salts thereof, methods of synthesis, and usage as more fully set forth below.

## Definitions

"Alkyl" means a branched or unbranched saturated hydrocarbon chain containing 1 to 10 carbon atoms, such as methyl, ethyl, propyl, iso-propyl, n-butyl, sec-butyl, tert-butyl, pentyl, n-hexyl, 2-methylheptyl, n-octyl and the like, unless otherwise indicated;

"Lower alkyl" means a branched or unbranched saturated hydrocarbon chain containing 1 to 4 carbon atoms, such as methyl, ethyl, propyl, isopropyl, tert-butyl, butyl and the like, unless otherwise indicated.

"Lower alkoxy" means the group -OR wherein R is lower alkyl as herein defined.

"Halo" as used herein denotes fluoro, chloro, bromo, or iodo, unless otherwise indicated.

"Phenyl" as used herein encompasses phenyl radicals optionally monosubstituted or disubstituted with a substituent selected from the group consisting of lower alkoxy, hydroxy, and halo.

"Optional" or "optionally" means that the subsequently described event or circumstance may or may

not occur, and that the description includes instances where said event or circumstance occurs and instances in which it does not. For example, "optionally substituted phenyl" means that the phenyl may or may not be substituted and that the description includes both unsubstituted phenyl and substituted phenyl.

"Phenethyl" means the phenylethyl radical of the formula $C_6H_5CH_2CH_2$- .

"Benzyl" means a phenyl group with a methylene group substituted on the ring.

"Aprotic polar solvent" as used herein includes organic solvents which may be either water-immiscible, such as halogenated hydrocarbons, e.g., methylene chloride, chloroform, etc., or water-miscible, such as tetrahydrofuran, dimethoxyethane, dimethylformamide, etc.

"Protecting group" means any suitable chemical group that is commonly used in the practice of organic chemistry to modify one or more of the major functional groups in a molecule for the purpose of selectively performing a chemical reaction at another reactive site in a multifunctional molecule. A protecting group is typically formed in a selective manner, is stable to subsequent reactions on the molecule and is selectively removed by reagents that do not attack the regenerated functional group. Suitable protecting groups for the hydroxy group are lower alkyl groups. Suitable protecting groups for the amino group are carbamates such as methyl carbamates and its derivatives like cyclopropylmethyl, diisopropylmethyl, 9-fluorenylmethyl carbamates and the like; substituted ethyl carbamates such as 2,2,2-trichloroethyl, 2-haloethyl, and the like; substituted propyl and isopropyl carbamates such as 1,1-dimethylpropynyl, 1-methyl-1-phenylethyl and derivatives, isobutyl, t-butyl carbamate, t-amyl carbamate, vinyl and allyl carbamate, phenyl and substituted phenyl carbamate, benzyl carbamate and derivatives such as p-methoxybenzyl, 3,5-dimethoxybenzyl, o- and p-nitrobenzyl, halobenzyl, and the like; amides and their derivatives such as N-acetyl and derivatives like N-dichloracetyl, N-trifluoroacetyl, and the like, substituted N-propionyl derivatives such as N-3-phenylpropionyl and derivatives, N-o-nitrocinnamoyl and the like, cyclic imide derivatives such as N-phthaloyl, N-2,3-diphenylmaleoyl, and the like. Suitable protecting groups for the hydroxyl group include, for example, optionally substituted alkyl groups such as methyl, ethyl, propyl, etc., alkylene groups such as allyl, or optionally substituted benzyl, trityl and the like.

A "leaving group" means a group capable of being displaced by a nucleophile in a chemical reaction, for example chloro, bromo, iodo, sulfonate ester, sulfinate ester, carbamate and the like.

The term "pharmaceutically acceptable salt" refers to those salts which retain the biological effectiveness and properties of the free bases of the invention and which are not biologically or otherwise undesirable. These salts may be prepared from either inorganic acids such as hydrochloric acid, hydrobromic acid, sulfuric acid, nitric acid, phosphoric acid, and the like; or organic acids such as acetic acid, oxalic acid, propionic acid, glycolic acid, pyruvic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, and the like.

The compounds of this invention possess an asymmetric center and thus can be produced as mixtures of stereoisomers or as individual (*R*), (*S*) or (*R,S*) stereoisomers. The individual stereoisomers may be obtained by using an optically active starting material, by resolving a racemic or non-racemic mixture of an intermediate at some appropriate stage of the synthesis or by resolution of a racemic or non-racemic mixture of the compound of formula (I). It is understood that the individual (*R*), (*S*) or (*R,S*) stereoisomers as well as mixtures (racemic and non-racemic) of stereoisomers are encompassed by the scope of the present invention.

In connection with the present invention the (*R*) stereoisomer is preferred.

"LAH" is an abbreviation used for the compound lithium aluminum hydride.

"$BH_3$•DMS" is an abbreviation used for borane dimethyl sulfide.

"THF" is an abbreviation used for the compound tetrahydrofuran.

"Isomers" are different compounds that have the same molecular formula.

"Stereoisomers" are isomers that differ only in the way the atoms are arranged in space.

"Enantiomers" are a pair of stereoisomers that are non-superimposable mirror images of each other.

"Diastereoisomers" are stereoisomers which are not mirror-images of each other.

"Epimers" are diastereoisomers which differ only in the configuration of one asymmetric center.

"Racemic mixture" means a mixture containing equal parts of individual enantiomers. "Non-racemic mixture" is a mixture containing unequal parts of individual enantiomers or stereoisomers.

The term "treatment" as used herein covers any treatment of a disease and/or condition in a mammal, particularly a human, and includes:

(i) preventing a disease and/or condition from occurring in a subject which may be predisposed to the disease and/or condition but has not yet been diagnosed as having it;

(ii) inhibiting the disease and/or condition, i.e., arresting its development; or

(iii) relieving the disease and/or condition, i.e., causing regression of the disease and/or condition.

Systems used in naming compounds of the present invention are shown below, using a compound of formula (I) as an example.

The compounds of formula (I) are named and numbered as illustrated below. For example a racemic mixture of compounds of formula (I) ((*R*) and (*S*) stereoisomers) wherein R is propyl, $R^1$ is hydroxy, m is 4, and n is 5 is shown as:

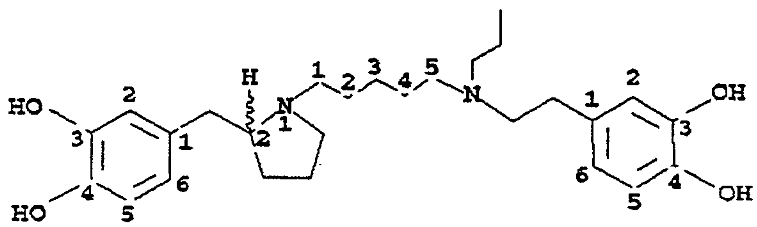

(I)

and named: (*R,S*)-2-(3,4-dihydroxybenzyl)-1-[5-(N-(3,4-dihydroxyphenethyl)-N-propylamino)pentyl]-pyrrolidine.

A compound of formula (I) wherein R is propyl, $R^1$ is hydroxy, m is 4, and n is 6 is named: (*R,S*)-2-(3,4-dihydroxybenzyl)-1-[6-(N-(3,4-dihydroxyphenethyl)-N-propylamino)hexyl]pyrrolidine.

A stereoisomer of a hydrobromide salt of a compound of formula (I) wherein R is propyl, $R^1$ is hydroxy, m is 3, and n is 6 is named: (R)-2-(3,4-dihydroxybenzyl)-1-[6-(N-(4-hydroxyphenethyl)-N-propylamino)-hexyl]pyrrolidine dihydrobromide.

A hydrochloride salt of a compound of formula (I) wherein R is propyl, $R^1$ is hydroxy, m is 3, and n is 5 is named: (R)-2-(3,4-dihydroxybenzyl)-1-[5-(N-(3-hydroxyphenethyl)-N-propylamino)pentyl]pyrrolidine dihydrochloride.

## REPRESENTATIVE COMPOUNDS

Representative compounds of formula (I) and salts of such compounds are given below. These compounds and their (*R*) or (*S*) stereoisomers or racemic mixtures thereof can be produced by the process steps described above. The (*R*) compounds are made by using (*R*)-proline as the starting material. The (*S*) compounds and racemic mixtures are made using (*S*)-proline as the starting material or a racemic mixture of (*R*) and (*S*)-proline, respectively. The (*R*)-proline gives the (*R*) compounds of formula (I) and (*S*)-proline gives the (*S*) compounds.

(*R*)-2-(3,4-dihydroxybenzyl)-1-[5-(N-(3,4-dihydroxyphenethyl)-N-propylamino)pentyl]pyrrolidine dihydrobromide

(*R*)-2-(3,4-dihydroxybenzyl)-1-[6-(N-(3,4-dihydroxyphenethyl)-N-propylamino)hexyl]pyrrolidine

(*R*)-2-(3,4-dihydroxybenzyl)-1-[5-(N-(3,4-dihydroxyphenethyl)-N-propylamino)pentyl]pyrrolidine dihydrobromide, $[\alpha]_D^{25}$ = -4.25

(*S*)-2-(3,4-dihydroxybenzyl)-1-[5-(N-(3,4-dihydroxyphenethyl)-N-propylamino)pentyl]pyrrolidine dihydrobromide, $[\alpha]_D^{25}$ = +2.12

(*R*)-2-(3,4-dihydroxybenzyl)-1-[6-(N-(3,4-dihydroxyphenethyl)-N-propylamino)hexyl]pyrrolidine dihydrobromide, $[\alpha]_D^{25}$ = -5.86

(*R,S*)-2-(3,4-dihydroxybenzyl)-1-[6-(N-(3,4-dihydroxyphenethyl)-N-propylamino)hexyl]pyrrolidine dihydrobromide, 471 (m+1, DCI)

(*S*)-2-(3,4-dihydroxybenzyl)-1-[6-(N-(3,4-dihydroxyphenethyl)-N-propylamino)hexyl]pyrrolidine dihydrobromide, $[\alpha]_D^{25}$ = +5.68

(*R*)-2-(3,4-dihydroxybenzyl)-1-[5-(N-(3,4-dihydroxyphenethyl)-N-butylamino)pentyl]pyrrolidine,

(*R*)-2-(3,4-dihydroxybenzyl)-1-[6-(N-(3,4-dihydroxyphenethyl)-N-butylamino)hexyl]pyrrolidine,

(*R*)-2-(3,4-dihydroxybenzyl)-1-[6-(N-(3,4-dihydroxyphenethyl)-N-butylamino)hexyl]pyrrolidine dihydrobromide,

(*R*)-2-(3,4-dihydroxybenzyl)-1-[5-(N-(4-hydroxyphenethyl)-N-propylamino)pentyl]pyrrolidine dihydrobromide, $[\alpha]_D^{25}$ = -5.14,

(*R*)-2-(3,4-dihydroxybenzyl)-1-[5-(N-(3-hydroxyphenethyl)-N-propylamino)pentyl]pyrrolidine,

(*R*)-2-(3,4-dihydroxybenzyl)-1-[5-(N-(4-hydroxyphenethyl)-N-propylamino)pentyl]pyrrolidine dihydrobromide,

(*R*)-2-(3,4-dihydroxybenzyl)-1-[5-(N-(3-hydroxyphenethyl)-N-propylamino)pentyl]pyrrolidine dihydrobromide, $[\alpha]_D^{25}$ = -1.15,

(*S*)-2-(3,4-dihydroxybenzyl)-1-[5-(N-(3-hydroxyphenethyl)-N-propylamino)pentyl]pyrrolidine dihydrobromide, $[\alpha]_D^{25}$ = +2.12

(*R*)-2-(3,4-dihydroxybenzyl)-1-[7-(N-(3,4-dihydroxyphenethyl)-N-propylamino)heptyl]pyrrolidine,

(*R*)-2-(3,4-dihydroxybenzyl)-1-[6-(N-(4-hydroxyphenethyl)-N-propylamino)hexyl]pyrrolidine, mp 113.5°-116.5° C

(*R*)-2-(3,4-dihydroxybenzyl)-1-[6-(N-(4-hydroxyphenethyl)-N-propylamino)hexyl]pyrrolidine dihydrobromide, $[\alpha]_D^{25}$ = -4.36

(*R*)-2-(3,4-dihydroxybenzyl)-1-[6-(N-(4-hydroxyphenethyl)-N-propylamino)hexyl]pyrrolidine dihydrochloride, mp 70.5°-73° C,

(*R*)-2-(3,4-dihydroxybenzyl)-1-[6-(N-(4-hydroxyphenethyl)-N-propylamino)hexyl]pyrrolidine dimaleate, mp 147°-149.5° C,

(*R*)-2-(3,4-dihydroxybenzyl)-1-[5-(N-(3,4-dihydroxyphenethyl)-N-propylamino)pentyl]pyrrolidine dihydrochloride, $[\alpha]_D^{25}$ = -5.42,

(*R*)-2-(3,4-dihydroxybenzyl)-1-[6-(N-(3,4-dihydroxyphenethyl)-N-propylamino)hexyl]pyrrolidine dihydrochloride,

(*R*)-2-(3,4-dihydroxybenzyl)-1-[5-(N-(4-hydroxyphenethyl)-N-butylamino)pentyl]pyrrolidine,

(*R*)-2-(3,4-dihydroxybenzyl)-1-[6-(N-(4-hydroxyphenethyl)-N-butylamino)hexyl]pyrrolidine,

(*R*)-2-(3,4-dihydroxybenzyl)-1-[6-(N-(4-hydroxyphenethyl)-N-butylamino)hexyl]pyrrolidine dihydrobromide, mp 162°-164° C, $[a]_D^{25}$ = -5.14

(*R*)-2-(3,4-dihydroxybenzyl)-1-[7-(N-(4-hydroxyphenethyl)-N-propylamino)heptyl]pyrrolidine,

(*R*)-2-(3,4-dihydroxybenzyl)-1-[6-(N-(3-hydroxyphenethyl)-N-propylamino)hexyl]pyrrolidine dihydrobromide, $[\alpha]_D^{25}$ = -2.91,

(*R,S*)-2-(3,4-dihydroxybenzyl)-1-[6-(N-(3-Hydroxyphenethyl)-N-propylamino)hexyl]pyrrolidine dihydrobromide, 454 (m+)

(*S*)-2-(3,4-dihydroxybenzyl)-1-[6-(N-(3-hydroxyphenethyl)-N-propylamino)hexyl]pyrrolidine dihydrobromide, mp 181°-183° C, $[\alpha]_D^{25}$ = +3.02

(*R*)-2-(3,4-dihydroxybenzyl)-1-[5-(N-(4-hydroxyphenethyl)-N-propylamino)pentyl]pyrrolidine dihydrochloride,

(*R*)-2-(3,4-dihydroxybenzyl)-1-[5-(N-(3-hydroxyphenethyl)-N-propylamino)pentyl]pyrrolidine dihydrochloride,

(*R*)-2-(4-hydroxybenzyl)-1-[5-(N-(3,4-dihydroxyphenethyl)-N-propylamino)pentyl]pyrrolidine,

(*R*)-2-(4-hydroxybenzyl)-1-[6-(N-(3,4-dihydroxyphenethyl)-N-propylamino)hexyl]pyrrolidine,

(*R*)-2-(3-hydroxybenzyl)-1-[5-(N-(3,4-dihydroxyphenethyl)-N-propylamino)pentyl]pyrrolidine dihydrobromide,

(*R*)-2-(3-hydroxybenzyl)-1-[6-(N-(3,4-dihydroxyphenethyl)-N-propylamino)hexyl]pyrrolidine dihydrobromide,

(*R*)-2-(4-hydroxybenzyl)-1-[5-(N-(3,4-dihydroxyphenethyl)-N-butylamino)pentyl]pyrrolidine,

(*R*)-2-(4-hydroxybenzyl)-1-[6-(N-(3,4-dihydroxyphenethyl)-N-butylamino)hexyl]pyrrolidine, and

(*R*)-2-(4-hydroxybenzyl)-1-[6-(N-(3,4-dihydroxyphenethyl)-N-butylamino)hexyl]pyrrolidine dihydrobromide.

It is to be understood that the terminology used herein is for the purpose of describing particular embodiments only, and is not intended to be limiting since the scope of the present invention will be limited only by the appended claims.

It must be noted that as used in this specification and the appended claims, the singular forms "a", "an" and "the" include plural referents unless the context clearly dictates otherwise. Thus, for example, reference to a dopamine agonist compound includes mixtures of such compounds, reference to "the hydrogenating reaction" includes reference to a plurality of hydrogenating reactions, reference to "an ester" includes mixtures of esters and so forth.

## METHODS OF PREPARATION

(Reaction Schemes I, II, and III)

Optically pure compounds such as the (*R*) stereoisomers of formula (I) of the present invention are prepared by using a commercially available optically pure starting material [(R)- and (S)-proline are commercially available from, e.g., Aldrich Chemical Company (Wisconsin) and Fluka Chemical Corporation] to prepare an optically pure intermediate (8) (Reaction Scheme I). Intermediate (8) is used as the starting material in Reaction Scheme III which results in the production of the optically pure (*R*) form of a compound of formula (I). The (*S*) form and racemic mixture can be obtained in the same manner by starting with the (*S*) form or racemic mixture respectively of the starting material.

REACTION SCHEME I

PREPARATION OF INTERMEDIATE (8)

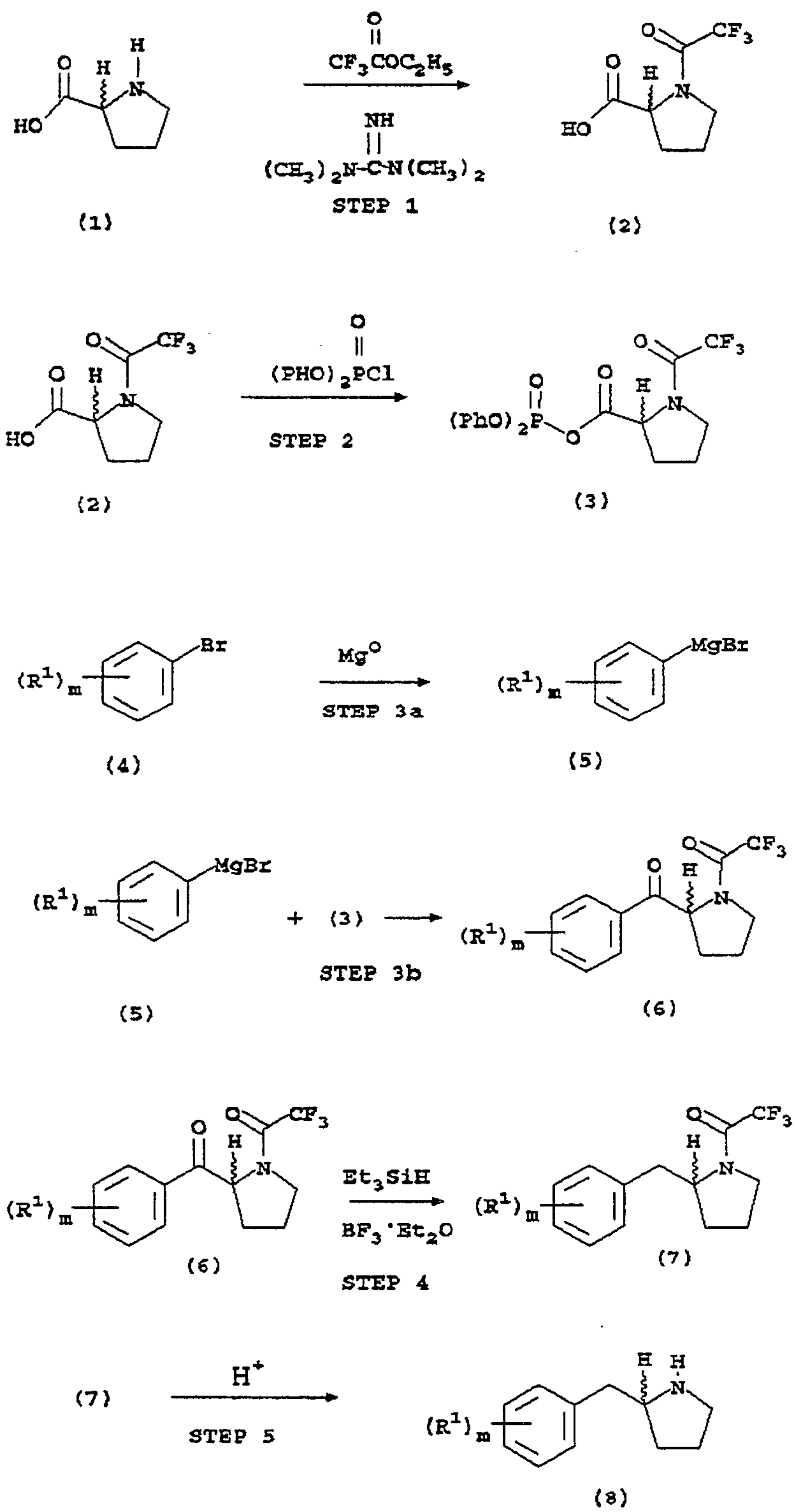


The instant invention is disclosed and described herein in what is considered to be the most practical, and preferred embodiments. It is recognized, however, that departures may be made therefrom which are within the scope of the invention, and that obvious modifications will occur to one skilled in the art upon reading this disclosure.

STEP 1 Scheme I

Formula 2

STEP (1) involves the protection of the amine function of (R)- or (S)-proline, preferably as the trifluoroacetyl derivative. Typically, the amine of formula (1) is reacted, in an inert solvent such as benzene, toluene, acetonitrile, diethyl ether, chloroform, methylene chloride or preferably tetrahydrofuran, with from 1 to 5 molar equivalents, preferably about 2 molar equivalents, of a trifluoroacetylating agent, preferably ethyl trifluoroacetate, in the presence of about 1.0 to 3 molar equivalents, preferably about 1.5 molar equivalents, of a tertiary organic base, such as pyridine, N-methylpiperidine, 4-dimethylaminopyridine and the like, preferably 1,1,3,3-tetramethylguanidine. The reaction is carried out at a temperature of about 0 to 40° C, preferably about 25° C, for about 10 minutes to 4 hours, preferably about 30 minutes. When the reaction is substantially complete, the product of formula (2), a 1-(trifluoroacetyl)- (R)- or (S)-proline is isolated by conventional means.

STEP 2 Scheme I

Formula 3

In STEP (2) the carboxyl group of the N-protected proline of formula (2) is converted to a derivative which on reaction with an organometallic compound gives a ketone. Methods for converting carboxyl groups to ketones are well known in the art, and include converting the carboxyl group to an acid halide and reacting this with a lithium dialkylcopper reagent or an organocadmium reagent, or converting the carboxyl group to a tertiary amide and reacting this with a Grignard reagent or organolithium derivative. Such reactions are discussed in more detail in Advanced Organic Chemistry by March, for example on pages 439-440 (2nd Edition), the pertinent portions of which are hereby incorporated by reference. The preferred method is to convert the carboxyl group to a mixed anhydride with diphenylphosphoric acid, most preferably to a (R)- or (S)-diphenylphosphoric-1- (trifluoroacetyl)-2-pyrrolidine carboxylic anhydride. Typically the protected amine of formula (2) is dissolved in an inert solvent as defined above, preferably methylene chloride, and reacted with from 0.5 to 1.5 molar equivalents, preferably about 1 molar equivalent, of an organochlorophosphate, preferably diphenyl chlorophosphate, in the presence of about 1.0 to 3 molar equivalents, preferably about 1.5 molar equivalents, of a tertiary organic base, such as pyridine, N-methylpiperidine and the like, preferably N-methylmorpholine. The reaction is carried out at a temperature of about -20 to 20° C, preferably about 0° C, for about 5 minutes to 1 hour, preferably about 10 minutes followed by a temperature of about 0 to 30° C, preferably about 25° C, for about 5 minutes to 1 hour, preferably about 10 minutes. When the reaction is substantially complete, the product of formula (3) is isolated by conventional means. The (R)- or (S)-diphenylphosphoric-1-(trifluoroacetyl)-2-pyrrolidine carboxylic anhydride of formula (3) is hygroscopic and unstable to heat and moisture, and is therefore preferably used in the next step without delay.

STEP 3a Scheme I

Formula 5

In Step (3a) a Grignard reagents of Formula 5 is prepared according to known methods. A bromobenzene derivative is added slowly to magnesium turnings in an aprotic polar solvent such as THF or diethylether at room temperature to reflux temperature of the solvent, preferably reflux temperature. A small

amount of iodine (one crystal) may be added to initiate the reaction. 1.1 to 1.5 molar equivalents of magnesium turnings are used. The reaction is generally complete in 1 to 6 hours.

STEP 3b Scheme I

Formula 6

In STEP (3b) the (R)- or (S)-2- diphenylphosphoric-1-(trifluoroacetyl)-2-pyrrolidine carboxylic anhydride of formula (3) is converted to a ketone of formula (6). Typically, the compound of formula (3) is dissolved in an inert solvent as defined above, preferably tetrahydrofuran, and cooled to a temperature of about -100 to -50° C, preferably about -60° C to -70° C. To the cold solution is added from 1 to 2 molar equivalents, preferably about 1.1 molar equivalents, of a Grignard reagent of formula (5) at such a rate that the temperature is maintained within the preferred range. The temperature of the reaction mixture is then allowed to rise to about 0 to 30° C, preferably about 25° C, for about 5 to 30 hours, preferably about 14 hours. When the reaction is substantially complete, the product of formula (6), (R)- or (S)-1-(trifluoroacetyl)-2-(3,4-dimethoxybenzoyl)-pyrrolidine, is isolated and purified by conventional means, preferably chromatography.

STEP 4 Scheme I

Formula 7

In STEP (4) the ketone of formula (6) is reduced to the compound of formula (7), (R)- or (S)-1-(trifluoroacetyl)-2-(3,4-dimethoxybenzyl)-pyrrolidine. Typically the compound of formula (6) is dissolved in an inert solvent as defined above, preferably methylene chloride. To the solution is added from 5 to 20 molar equivalents, preferably about 10-12 molar equivalents, of a reducing agent, preferably triethylsilane/boron trifluoride etherate. The reaction is carried out at a temperature of about 0 to 30° C, preferably about 25° C, for about 1 to 7 days, preferably about 3 days. When the reaction is substantially complete, the product of formula (7) is isolated by conventional means.

STEP 5 Scheme I

Formula 8

In STEP (5), the amine protecting group is removed from the compound of formula (7). Typically the compound of formula (7) is dissolved in a protic solvent such as ethanol, n-propanol, n-butanol, t-butanol and the like, preferably isopropanol, and to the solution is added from 5 to 50 molar equivalents, preferably about molar equivalents, of an acid, for example sulfuric acid, HBr and the like, or preferably 12.5 M hydrochloric acid. The reaction is carried out at the reflux temperature of the solvent chosen, preferably about 70 to 90° C, for about 8 to 48 hours, preferably about 24 hours. When the reaction is substantially complete, the product of formula (8), (R)- or (S)-2-(3,4-dimethoxybenzyl)pyrrolidine, is isolated by conventional means.

REACTION SCHEME II

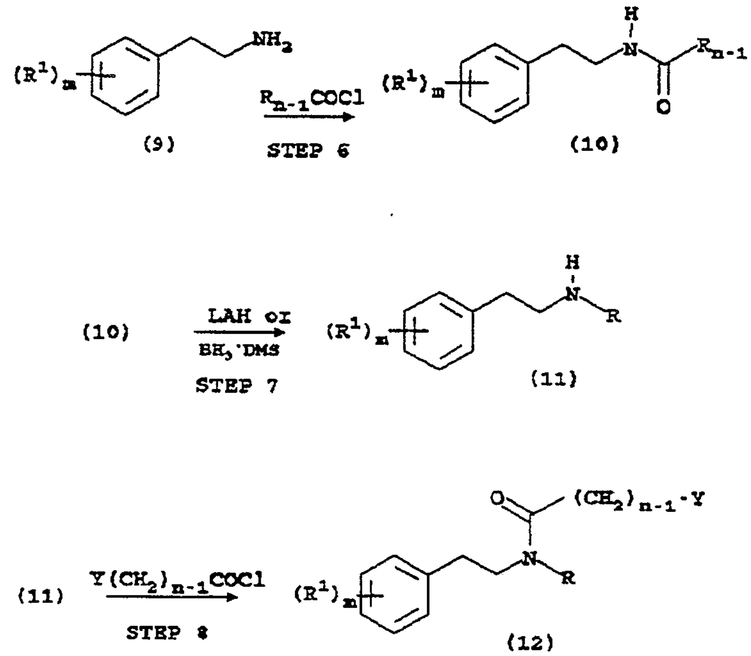


NOTE: The symbols above are defined following Reaction Scheme III

STEP 6 Scheme II

Formula 10

In Step (6) intermediates of formula (10) (wherein $R_{n-1}$ is an alkyl chain of 1, 2, or 3 carbon atoms) are prepared by reacting a phenethylamine with an acyl halide in the presence of a tertiary organic base (e.g., triethylamine, pyridine) in a suitable inert solvent (e.g., dichloromethane). 1 to 1.3, preferably 1.1 to 1.2, molar equivalents of acyl halide and 1.5 to 3, preferably 2 to 2.5, molar equivalents of base are used. The reaction is carried out at -10° C to 30° C, preferably 0° C to 20° C. Reaction time is generally 1 to 4 hours. The reaction product is isolated and purified by conventional means, e.g., extraction, crystallization, or the like.

STEP 7 Scheme II

Formula 11

In Step (7) intermediate (10) is reduced to the N-alkylated phenethylamine intermediate (11) with, for example, lithium aluminum hydride (LAH) or borane-methyl sulfide complex ($BH_3 \cdot DMS$). Typically, the amide (intermediate 10) is dissolved in THF and added dropwise to a solution of LAH in THF at 25° to 70° C, preferably 40° to 60° C. 1 to 1.5, preferably 1.2 to 1.3 molar equivalents of LAH are used. The reaction mixture is heated under reflux for 1 to 6 hours, preferably 2 to 4 hours. The reaction product is

isolated by conventional means, e.g., filtration, evaporation of solvent, or the like.

Alternatively, the amide (intermediate 10) is dissolved in dry THF and added to a solution of 2 to 10, preferably 5 molar equivalents of borane-methyl sulfide complex ($BH_3$.DMS) in THF. The reaction mixture is heated under reflux for 1 to 4, preferably 2 hours, under argon. After cooling to room temperature, methanol is added dropwise followed by saturated HCl-MeOH. The reaction mixture is heated for 10 to 30 minutes, preferably 15 to 20 minutes, and then allowed to cool to room temperature. The solvents are evaporated under reduced pressure. The residue is dissolved in isopropanol and diethyl ether is added until the solution becomes slightly cloudy. The product (intermediate 11) generally crystallizes out in 10 to 20 hours at room temperature.

Intermediate 11 wherein R is methyl can be prepared by either of two methods: (1) reacting intermediate 9 with formyl anhydride, followed by reduction with borane, or (2) converting an appropriately substituted phenylacetic acid to the corresponding acid chloride, then treating the acid chloride with methylamine to form the amide, followed by reducing the amide with borane to form intermediate 11 wherein R is methyl. Compound of Formula I wherein R is methyl can be prepared using intermediate 11 wherein R is methyl and following Reaction Schemes II and III.

STEP 8 Scheme II

Formula 13

In Step (8) intermediate (11) is acylated with an ω-haloalkanoyl chloride such as 6-bromohexanoyl chloride in the presence of an inorganic base (e.g., potassium carbonate) under aqueous conditions. Typically, the ω-haloalkanoyl chloride is added dropwise to a solution of N-alkylphenethylamine hydrochloride and $K_2CO_3$ in water and ethyl acetate. 1 to 1.6, preferably 1.3 to 1.4 molar equivalents of ω-haloalkanoyl chloride and 1.2 to 1.8, preferably 1.5 molar equivalents of $K_2CO_3$ are used. The reaction is carried out at 0° to 30° C, preferably 0° C to 10° C. Reaction time is generally 1 to 5 hours, preferably 2 to 3 hours. The reaction product (intermediate 12) is isolated by conventional means, e.g., extraction, filtration, evaporation, or the like.

Compounds of Formula I wherein R is hydrogen can be prepared by reacting intermediate (9) in Step 8 (instead of intermediate (11)). Reaction Scheme III is then followed to prepare compounds of Formula I wherein R is hydrogen.

## REACTION SCHEME III

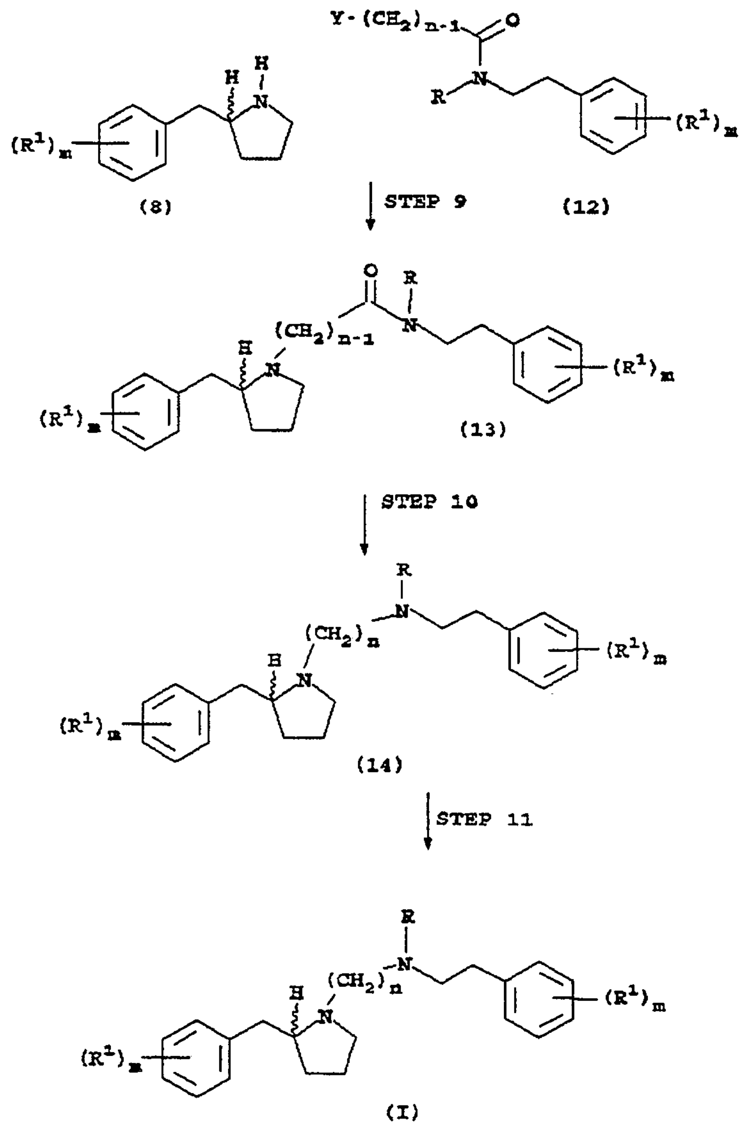


(8) (12)

(13)

(14)

(I)

Definition of Symbols

Y is halo

R, $R^1$, m and n are as defined above

### STEP 9 Scheme III

### Formula 13

In Step (9) intermediates of formula 13 are produced by condensing a 2-benzylpyrrolidine of formula 8 with an N-(ω-haloalkanoyl)phenethylamine of formula 12. Typically, the condensation is carried out in an aprotic polar solvent (e.g., dimethylformamide) using equimolar amounts of the intermediate (8) and (12) in

the presence of 2 to 5, preferably 3 to 4 molar equivalents of a tert.organic base (e.g., triethylamine) and about 1 molar equivalent of an inorganic base (e.g., $K_2CO_3$) and a small amount of NaI. Reaction temperature ranges from room temperature to 100°, preferably 50 to 70° C. The reaction time is generally 6 to 24 hours. The reaction product (intermediate 13) is isolated by conventional means, e.g., extraction, evaporation, or the like.

STEP 10 Scheme III

Formula 14

In Step (10) the carbonyl group of intermediate (13) is reduced to the hydrocarbon (intermediate 14) following known procedures (see Step 7 above). Intermediate (14) is isolated by conventional means and further purified by chromatography on silica gel.

STEP 11 Scheme III

Formula I

In Step (11) compounds of formula I are obtained by demethylating intermediates of formula (14) according to known procedures. Typically, the reaction is carried out in an inert solvent such as dichloromethane at 0° to room temperature under nitrogen. An excess of 1M $BBr_3$ or $BCl_3$ in $CH_2Cl_2$ (4 to 10, preferably 6 to 8 molar equivalents) is used. When the reaction is substantially complete by tlc the solvents are evaporated under reduced pressure. Further purification by recrystallization yields the compound of formula I as the dihydrobromide or dihydrochloride salt.

Compounds of Formula I wherein $R^1$ is other than -OH may be prepared according to the following methods. For example, 4-methoxybromobenzene is converted to 2-(4-methoxybenzyl)pyrrolidine according to Steps 1 to 5 (Reaction Scheme I). 2-(4-methoxybenzyl)pyrrolidine may then be nitrated with a mixture of conc. nitric acid in acetic acid in the presence of a catalytic amount of conc. sulfuric acid at 0° to 5° C. 2-(3-nitro-4-methoxybenzyl)pyrrolidine is isolated and purified by conventional means, e.g., extraction, chromatography, cyrstallization, or the like.

2-(3-nitro-4-methoxybenzyl)pyrrolidine is then condensed with an intermediate of Formula 12 to produce a compound of Formula 13 which is reduced to a compound of Formula 14 with a reducing agent such as boranemethylsulfide complex ($BH_3$•DMS) according to Steps 9 and 10 (Reaction Scheme III). The 2-(3-amino-4-methoxybenzyl)pyrrolidine intermediate of Formula 14 may then be transformed to a number of amide derivatives according to methods known to those skilled in the art.

For example, intermediate 14 may be treated with methanesulfonyl chloride in $CH_2Cl_2$ in the presence of a tertiary organic base such as triethylamine to produce a 2-(3-methylsulfonylamino-4-methoxybenzyl) pyrrolidine intermediate of Formula 14. Demethylation with, for example, $BB_3$ or $BCl_3$ in $CH_2Cl_2$ yields the corresponding 2-(3-methylsulfonylamino-4-hydroxybenzyl) pyrrolidine compound of Formula I as the dihydrobromide or dihydrochloride salt, respectively (Step 11, Reaction Scheme III).

UTILITY AND ADMINISTRATION

The compounds of Formula I and the pharmaceutically acceptable non-toxic esters and salts thereof, are useful in the treatment of hypertension, congestive heart failure, acute and chronic renal failure, angina and hyperprolactenemia in mammals. These compounds can be used both prophylactically and therapeutically.

Pharmaceutical dosage forms which include compositions containing compounds of formula (I) and

salts thereof are thus administered to patients suffering from hypertension or congestive heart failure. The compounds act to relieve blood pressure and improve heart action by acting as dopamine agonists. In addition, these compositions may be used to treat other conditions as recognized by those skilled in the art.

Administration of the active compounds and salts described herein can be via any of the accepted modes of administration for cardio regulating agents. These methods include oral and intravenous modes, preferably oral administration. Intravenous administration would preferably be reserved for crisis situations, wherein the subject is unable to swallow or administer the medication to himself.

Depending on the intended mode, the compositions may be in the form of solid, semi-solid or liquid dosage forms, such as, for example, tablets, pills, capsules, powders, liquids, suspensions, or the like, preferably in unit dosage forms suitable for single administration of precise dosages. The compositions will include a conventional pharmaceutical carrier or excipient and an active compound fo Formula I or the pharmaceutically acceptable salts thereof and, in addition, may include other medicinal agents, pharmaceutical agents, carriers, adjuvants, etc.

The amount of active compound administered will, of course, be dependent on the subject being treated, the severity of the affliction, the manner of administration and the judgment of the prescribing physician. However, an effective dosage is in the range of 0.001 to 50 mg/kg/day, preferably 0.01 to 30 mg/kg/day. For an average 70 kg human, this would amount to 0.07 to 3500 mg per day, or preferably 0.7 to 2100 mg/day.

For solid compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like may be used. The active compound as defined above may be formulated as suppositories using, for example, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine oleate, etc. Actual methods of preparing such dosage forms are known, or will be apparent, to those skilled in this art; for example, see Reminaton's Pharmaceutical Sciences , Mack Publishing Company, Easton, Pennsylvania, 15th Edition, 1975. The composition or formulation to be administered will, in any event, contain a quantity of the active compound(s) in an amount effective to alleviate the symptoms of the subject being treated.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, glucose, sucrose, magnesium carbonate, and the like. Such compositions take the form of solutions, suspensions, tablets, pills, capsules, powders, sustained release formulations and the like. Such compositions may contain 0.1% to 95% active ingredient, preferably 1% to 70%.

For intravenous injections, the compound is dissolved in aqueous medium, buffered to the proper pH and formed to control isotonicity for injection.

Initially, compounds of general structural formula (I) wherein R is propyl, $R^1$ is OH, m is 3 or 4 and n is 5 or 6 were screened for affinity at the D1 and D2 dopamine receptor subtypes via ligand binding techniques. D1 and D2 dopamine receptors in rat striatal membranes were labelled with 0.2 nM [3H]SCH 23390 and 0.2 nM as that level of binding that was not displaced by 1 $\mu$M (+)butaclamol. [3H]Spiperone competition studies were done in the presence of 30 nM ketanserin. These compounds, which were examined at concentrations from 0.1 nM to 100 uM, demonstrated high affinity for the D1 and D2 receptor subtypes in these assays.

The compounds were subsequently tested following intra-arterial administration in an in situ renal and femoral arterial preparation in the dog, as developed by L.I. Goldberg, to test for DA1 and DA2 dopamine activity, respectively. Details of the methodology are provided in the European J. Pharmacol. 89 :137, 1983, as well as in Hypertension 6 :1-25, 1984. These compounds were found to be highly active when compared to dopamine and di-propyl dopamine, the respective DA1 and DA2 standards for this preparation.

Additionally, these compounds were examined for diuretic activity for 6 hours following oral compound administration in the saline-loaded spontaneously hypertensive rat (SHR). Methods for this assay have been previously described (Rosenkranz, et al, Proc. West. Pharmacol. Soc. 28 :87, 1985). The diuretic and natriuretic effects elicited by these compounds were characterized by an immediate onset of action. Over the course of the 6 hour study, these compounds were found to be as efficacious as the standard diuretic agent, hydrochlorothiazide.

Finally, these compounds were screened for oral antihypertensive activity in the conscious restrained rat. Adult male SHR were instrumented for blood pressure and heart rate measurements under light ether anesthesia. The animals were maintained on a plexiglass restraining board following surgery, and were allowed a 1 hour recovery period prior to the oral administration of the test compounds. These compounds were found to decrease blood pressure in the absence of a reflex tachycardia for up to 4 hours post-dosing.

## EXAMPLE 1

(Reaction Scheme 1)

((*R*)-1-(trifluoroacetyl)proline (Compound 2))

In a 500 ml round bottom flask was placed D-proline (20 g, 0.174 moles). To this was added dry THF (100 ml) and ethyl trifluoroacetate (50 g, 0.35 moles). The solution/flask was purged with argon and 1,1,3,3-tetramethylguanidine (30 g, 0.261 moles) was added dropwise. The solution was allowed to stir until all the D-proline had dissolved (approximately 35 minutes). The solvent was removed in vacuo and the residue dissolved in $CH_2Cl_2$ (200 ml). The solution was washed with aqueous 6N HCl (2 x 100 ml). The organic layer was separated and dried with $Na_2SO_4$, filtered and the solvent removed in vacuo to give an oil which crystallized upon standing to yield: 29 g (79%) of (*R*)-1-(trifluoroacetyl)proline, mp, 48°-51° C.

## EXAMPLE 2

(*R*)-diphenylphosphoric-1-(trifluoroacetyl)-2-pyrrolidine carboxylic anhydride

(*R*)-1-(trifluoroacetyl)proline (compound 2) (29 g, 0.137 moles) was dissolved in $CH_2Cl_2$ (300 ml) in a 1 liter round bottom flask and cooled to 0° C. To this solution was added diphenyl chlorophosphate (36.9 g, 0.137 moles) followed by 4-methylmorpholine (15.27 g, 0.151 moles). After stirring at 0° C for 10 minutes, the reaction mixture was allowed to warm to room temperature and stir for an additional 10 minutes. The solution was then diluted with 600 ml dry diethyl ether and filtered. The filtrate was washed with a saturated solution of $NaHCO_3$, the organic layer was separated, dried over $MgSO_4$, filtered and the solvent removed in vacuo to give 54.8 g (90%) of (*R*)-diphenylphosphoric-1-(trifluoroacetyl)-2-pyrrolidine carboxylic anhydride (intermediate 3), as a solid which was hygroscopic and unstable to heat and moisture.

## EXAMPLE 3

(*R*)-1-(trifluoroacetyl)-2-(3,4-dimethoxybenzoyl)pyrrolidine

To a 250 ml round bottom flask was added Mg° turnings (4.51 g, 0.186 moles) and dry THF 100 ml. Iodine (one crystal) was added, followed by bromoveratrole 27 g, 0.124 moles) dropwise. After addition of the first few drops of bromoveratrole the reaction was heated under reflux until the iodine color disappeared. The remainder of 4-bromoveratrole was then added dropwise. A condition of reflux was maintained for 2 hours. The solution was then cooled to room temperature and added to a solution of (*R*)-diphenylphosphoric-1-(trifluoroacetyl)-2-pyrrolidine carboxylic anhydride (intermediate 3) (54 g, 0.123 moles) in dry THF (250 ml) at -70° C. The rate of addition was monitored to keep the reaction temperature below

-60° C. Once addition was complete the reaction mixture was warmed to room temperature and allowed to stir for 14 hours. It was then poured into a saturated solution of ammonium chloride (500 ml) and shaken in a separatory funnel. The organic layer was separated and dried over $MgSO_4$, filtered, and the solvent removed in vacuo to give an oil. This oil was purified by flash chromatography eluting with hexane:ethyl acetate (1:1) to give 20.4g (50%) of (*R*)-1-(trifluoroacetyl)-2-(3,4-dimethoxybenzoyl)pyrrolidine (intermediate 6) mp 121°-123° C, $[\alpha]_D^{25}$ = +65°

## EXAMPLE 4

### (*R*)-1-(trifluoroacetyl)-2-(3,4-dimethoxybenzyl)pyrrolidine

(*R*)-1-(trifluoroacetyl)-2-(3,4-dimethoxybenzoyl)pyrrolidine (intermediate 6) (4.9 g, 0.015 moles) was dissolved in dry $CH_2Cl_2$ (50 ml) in a 500 ml round bottom flask. To the solution was added triethylsilane (20 g, 0.172 moles) and $BF_3 \bullet Et_2O$ (50 ml). The reaction mixture was stirred at room temperature for 3 days, after which time a saturated solution of potassium carbonate was added cautiously in a dropwise manner until all gas evolution had ceased. $CH_2Cl_2$ (100 ml) was added and the mixture was shaken in a separatory funnel. The triphasic mixture was filtered through a fritted glass funnel and the organic layer was separated and dried over $MgSO_4$. Removal of the solvent in vacuo yielded (*R*)-1-(trifluoroacetyl)-2-(3,4-dimethoxybenzyl)pyrrolidine as an oil (3.2 g, 68%) which was used without further purification.

## EXAMPLE 5

### (*R*)-2-(3,4-dimethoxybenzyl)pyrrolidine

To a solution of (*R*)-1-(trifluoroacetyl)-2-(3,4-dimethoxybenzyl)pyrrolidine (3.2 g, 0.010 moles) in isopropyl alcohol (50 ml) was added 12.5 M HCl (15 ml). The mixture was heated under reflux until the reaction was complete (approximately 24 hours). The solvent was removed in vacuo to give an oil which was recrystallized from isopropyl alcohol-ether to give (*R*)-2-(3,4-dimethoxybenzyl) pyrrolidine (intermediate 8), (2.5 g, 97%), m.p. 165°-167° C.

## EXAMPLE 6

### N-propionyl-4-methoxyphenethylamine

A mixture of 4-methoxyphenylethylamine (25 ml, 0.17 moles), triethylamine (25 ml), and methylene chloride (250 ml) was cooled to 0° C. To this solution was added propionyl chloride (17.4 ml, 0.2 moles) dropwise. The mixture was stirred for 2 hours at 0° to 20° C and was then washed with 1N HCl (300 ml) followed by saturated $NaHCO_3$ solution. The organic layer was dried over $Na_2SO_4$, filtered and the solvent removed under vacuum to give N-propionyl-4-methoxyphenethylamine (intermediate 10) (32.4 g), as a yellow solid. Recrystallization from $Et_2O$ gave a white solid, mp 114°-116° C.

## EXAMPLE 7

N-propyl-4-methoxyphenethylamine hydrochloride

N-propionyl-4-methoxyphenethylamine (intermediate 10) (32.4g) in THF (100 ml) was added dropwise to a warm (40-60° C) solution of LAH (6.5 g) in THF (200 ml). After the addition the solution was heated under reflux for 3 hours. The mixture was then cooled to room temperature, the excess LAH destroyed by the sequential addition of water (6.5 ml), 10% NaOH (7 ml), and water (20 ml). The precipitate was removed by filtration, the filter cake washed with diethyl ether, and the filtrate concentrated under vacuum to give a thick oil. The oil was dissolved in isopropanol and acidified with methanolic HCl. Diethyl ether was added, and N-propyl-4-methoxyphenethylamine hydrochloride (intermediate 11) which precipitated out of the solution, was filtered and air dried to give 22 g of a white solid, m.p. 197° -200° C.

## EXAMPLE 8

N-propyl-N-(6-bromohexanoyl)-4-methoxyphenethylamine

To a cold (0-5° C) solution of N-propyl-4-methoxyphenethylamine hydrochloride (11 g, 48 mmoles), $K_2CO_3$ (10 g), $H_2O$ (200 ml), and ethyl acetate (300 ml) was added dropwise 6-bromohexanoyl chloride (10 ml, 65 mmoles). The mixture was stirred for 2 hours. The layers were then separated using a separatory funnel, and the organic layer dried over $Na_2SO_4$. The drying agent was removed by filtration. The solvent was evaporaterd under vacuum to give 14 g (78%) N-propyl-N-(6-bromohexanoyl)-4-methoxyphenethylamine (intermediate 12) as a yellow oil.

## EXAMPLE 9

(*R*)-2-(3,4-dimethoxybenzyl)-1-[5-(N-(4-methoxyphenethyl)-N-propylamino)carbonylpentyl]pyrrolidine

(*R*)-2-(3,4-dimethoxybenzyl)pyrrolidine (intermediate 8) (2 g), N-propyl-N-(6-bromohexanoyl)-4-methoxyphenethylamine (intermediate 12) (3 g), triethylamine (5 ml), $K_2CO_3$ (1 g), and Hal (0.1 g) were added to DMF (50 ml). The mixture was heated at 60° C for 18 hours. The mixture was then poured into ice water (200 ml). The solution was acidified with conc. HCl and washed with $Et_2O$. The aqueous layer was separated and basified with 10% NaOH solution and the product extracted with ethyl acetate. The organic layer was dried over $Na_2SO_4$, and the solvent evaporated to yield 2.7 g of (*R*)-2-(3,4-dimethoxybenzyl)-1-[5-(N-(4-methoxyphenethyl)-N-propylamino)carbonylpentyl]pyrrolidine (intermediate 13).

## EXAMPLE 10

(*R*)-2-(3,4-dimethoxybenzyl-1-[6-(N-(4-methoxyphenethyl)-N-propylamino)hexyl]pyrrolidine

(*R*)-2-(3,4-dimethoxybenzyl)-1-[5-(N-(4-methoxyphenethyl)-N-propylamino)carbonylpentyl]pyrrolidine (intermediate 13) was dissolved in THF (20 ml) and added slowly to a refluxing solution of LAH (1 g) in THF (150 ml). The solution was heated under reflux for an additional 3 hours and then cooled to room temperature. Excess LAH was destroyed by careful addition of $H_2O$. After filtration, the solvent was evaporated under vacuum to give crude compound (14) as a thick oil. Chromatography on silica gel with 3% MeOH in $CH_2Cl_2$ gave 1 g of (*R*)-2-(3,4-dimethoxybenzyl-1-[6-(N-(4-methoxyphenethyl)-N-propylamino)-hexyl]pyrrolidine (intermediate 14).

EXAMPLE 11

(*R*)-2-(3,4-dihydroxybenzyl)-1-[6-(N-(4-hydroxyphenethyl)-N-propylamino)hexyl]pyrrolidine dihydrobromide

(R)-2-(3,4-dimethoxybenzyl-1-[6-(N-(4-methoxyphenethyl)-N-propylamino)hexyl]pyrrolidine (intermediate 14) (1 g) was dissolved in $CH_2Cl_2$ (20 ml) and cooled to 0° C, under $N_2$. An excess of 1M $BBr_3$ (15 ml) in $CH_2Cl_2$ was added and the mixture allowed to warm to room temperature. When the reaction was complete by TLC, the solution was cooled to 0° C and MeOH was added. The solvents were then removed under vacuum, MeOH was again added and the solvent evaporated at 40° C under vacuum and dried for 18 hours at room temperature under vacuum. The resulting pink foam (0.75 g, 61%) was (*R*)-2-(3,4-dihydroxybenzyl)-1-[6-(N-(4-hydroxyphenethyl)-N-propylamino]hexyl]pyrrolidine dihydrobromide (compound (I)). $[\alpha]_D^{25}$ = -5.14.

EXAMPLE 12

(R)-2-(3,4-dihydroxybenzyl)-1-[6-(N-(4-hydroxyphenethyl)-N-propylamino) hexyl]pyrrolidine dihydrochloride

(R)-2-(3,4-dimethoxybenzyl)-1-[6-(N-(4-methoxyphenethyl)-N-propylamino)hexyl]pyrrolidine (1g) was dissolved in dry $CH_2Cl_2$ (4 ml) and placed in a round bottom flask. One molar boron trichloride in $CH_2Cl_2$ (8 molar equivalents) was added rapidly with stirring. The reaction mixture was then heated under gently reflux for hours. The reaction mixture was cooled to room temperature and anyhdrous methanol (10 ml) was carefully added. The solution was heated under reflux for 45 minutes and the solvents were then removed under reduced pressure. Anydrous methanol (10 ml) was added, followed by removal of the solvent under reduced pressure (2 times). The residue was dried under vacuum at 40° C to 50° C for 3 hours. Recrystallization from methanol/ethyl acetate gave 0.69 g (86%) of (R)-2-(2,3-dihydroxybenzyl)-1-[6-(N-(4-hydroxyphenethyl)-N-propylamino)hexyl]pyrrolidine dihydrochloride as a white solid, m.p. 70.5-73° .

EXAMPLE 13A

Salt to free base

To a 3 L round bottom flask containing (R)-2-(3,4-dihydroxybenzyl)-1-[6-(N-(4-hydroxyphenethyl)-N-propylamino)hexyl]pyrrolidine dihydrobromide (14.5g), water (600 ml), ethyl acetate (1200 ml), and methanol (25 ml) was added dropwise with stirring a saturated solution of sodium bicarbonate (175 ml). After completion of the addition, the organic phase (EtOAc) containing most of the free base was separated. The aqueous phase was extracted with ethyl acetate. The organic extracts were then combined and dried over $MgSO_4$. Evaporation of the solvent under reduced pressure gave the free base as a viscous oil which solidified to a beige solid (10.69 g, quantitative yield), m.p. 113.5-116° C.

EXAMPLE 13B

Free Base to Salt

(R)-2-(3,4-dihydroxybenzyl)-1-[6-(N-(4-hydroxyphenethyl)-N-propylamino)hexyl]pyrrolidine . (2g, 4.4

mmol) was dissolved in methanol (5 ml) and then added to a solution of maleic acid (1.02g, 8.8 mmol) in methanol (5 ml). The solution was concentrated to a minimum volume of methanol. Diethyl ether was added until the solution turned cloudy. The solution was allowed to stand at room temperature for about 20 hours to yield a white solid which was collected by filtration and dried at 40° C to 50° C under nitrogen to give 2.75g (91%) of (R)-2-(3,4-dihydroxybenzyl)-1-[6-(N-(4-hydroxyphenethyl)-N-propylamino)hexyl]pyrrolidine dimaleate, m.p. 147-149.5° C.

## EXAMPLE 14

### Conversion from Salt to Salt

To a 3 L round bottom flask containing (R)-2-(3,4-dihydroxybenzyl)-1-[6-(N-(4-hydroxyphenethyl)-N-propylamino)hexyl]pyrrolidine dihydrobromide (14.5g), water (600 ml), ethyl acetate (1200 ml), and methanol (25 ml) was added dropwise with stirring a saturated solution of sodium bicarbonate (175 ml). After completion of the addition, the organic phase (EtOAc) containing most of the free base was separated. The aqueous phase was extracted with ethyl acetate. The organic extracts were then combined and dried over $MgSO_4$. Evaporation of the solvent under reduced pressure gave a residue which was dissolved in methanol (5 ml) and then added to a solution of maleic acid (1.02g, 8.8 mmol) in methanol (5 ml). The solution was concentrated to a minimum volume of methanol. Diethyl ether was added until the solution turned cloudy. The solution was allowed to stand at room temperature for about 20 hours to yield a white solid which was collected by filtration and dried at 40° C to 50° C under nitrogen to give 2.75g (91%) of (R)-2-(3,4-dihydroxybenzyl)-1-[6-(N-(4-hydroxyphenethyl)-N-propylamino)hexyl]pyrrolidine dimaleate, m.p. 147-149.5° C.

## EXAMPLE 15

### Goldberg Renal and Femoral *In Vivo* Dog Model

The compounds of this invention were tested following intra-arterial administration in an *in situ* renal and femoral arterial preparation in the dog, as developed by L.I. Goldberg, to test for DA1 and DA2 dopamine activity, respectively. Details of the methodology are provided in the European J. Pharmacol. , 89 :137, 1983, as well as in Hypertension , 6 :1-25, 1984. J. L. McNay, L. I. Goldberg JPET 151 :23-31, 1966.

The compounds were found to be highly active when compared to dopamine and di-propyl dopamine, the respective DA1 and DA2 standards for this preparation.

The compounds were injected directly into the renal or femoral artery for analysis of $DA_1$ or $DA_2$ activity, respectively. Since the compounds were introduced into the $DA_1$ and $DA_2$ receptor beds in minimal doses, specific renal and femoral vasodilation was observed in the absence of systemic effects. The compounds were tested for relative activity in comparison to the standard $DA_1$ agonist, dopamine or the standard $DA_2$ agonist, DPDA. Specific $DA_1$ or $DA_2$ activity of the tested compound was verified by Sch 23390 or domperidone blockade.

Adult mongrel dogs of either sex (12-20 kg) were anesthetized with pentobarbital sodium (33 mg/kg, i.v.). The animals were intubated and ventilated with room air using a Harvard respirator.

### Renal

The abdominal aorta was catheterized with a Millar aqueous phase was extracted with ethyl acetate. The organic extracts were then combined and dried over $MgSO_4$. Evaporation of the solvent under reduced pressure gave a residue which solidified to a beige solid (10.69g, quantitative yield), m.p. 113.5-116° C.

## EXAMPLE 15

### Goldberg Renal and Femoral In Vivo Dog Model

The compounds of this invention were tested following intra-arterial administration in an in situ renal and femoral arterial preparation in the dog, as developed by L.I. Goldberg, to test for DA1 and DA2 dopamine activity, respectively. Details of the methodology are provided in the European J. Pharmacol. , 89 :137, 1983, as well as in Hypertension , 6 :1-25, 1984. J. L. McNay, L. I. Goldberg JPET 151 :23-31, 1966.

The compounds were found to be highly active when compared to dopamine and di-propyl dopamine, the respective DA1 and DA2 standards for this preparation.

The compounds were injected directly into the renal or femoral artery for analysis of $DA_1$ or $DA_2$ activity, respectively. Since the compounds were introduced into the $DA_1$ and $DA_2$ receptor beds in minimal doses, specific renal and femoral vasodilation was observed in the absence of systemic effects. The compounds were tested for relative activity in comparison to the standard $DA_1$ agonist, dopamine or the standard $DA_2$ agonist, DPDA. Specific $DA_1$ or $DA_2$ activity of the tested compound was verified by Sch 23390 or domperidone blockade.

Adult mongrel dogs of either sex (12-20 kg) were anesthetized with pentobarbital sodium (33 mg/kg, i.v.). The animals were intubated and ventilated with room air using a Harvard respirator.

#### Renal

The abdominal aorta was catheterized with a Millar micro-tip (size: 5F) transducer via the right femoral artery for blood pressure monitoring. The right femoral vein was cannulated with PE-160 tubing for iv saline infusion, as well as supplemental pentobarbital administration. The left renal artery was isolated through a lateral abdominal incision. An electromagnetic flowprobe of the appropriate size (8-11 mm circ.) was placed around the artery and connected to a Carolina Medical electromagnetic flowmeter. Surface ECG leads were placed subcutaneously on the chest and limbs to monitor heart rate. A 25 gauge 3/4" needle bent 90°, 4 mm from the tip, was placed in the renal artery proximal to the probe and connected to an infusion pump delivering saline at a constant rate of 1 ml/min. Following a saline and 1µg norepinephrine (NE) challenge, both given in an injection volume of 0.2 ml, the animal was treated with a phenoxybenzamine, 0.5 mg/kg/min i.a., infusion for a duration of 10-20 minutes as necessary to achieve a complete blockade of the NE challenge. Blood pressure was maintained by supplemental saline infusion (10-20 ml/kg, iv). Bradykinin, a standard for nonspecific vasodilation, was given at 0.60 nmole doses, in 0.2 ml saline, i.a., prior to generating a dose response curve for dopamine in which 4-fold increasing doses, ranging from 3 to 48 nmoles, were injected i.a. in 0.2 ml of saline (i.a. bolus). Test compounds were injected similarly in 4-fold increasing doses up to a maximum of 3000 nmoles. Compounds were then repeated at their optimal dose following Sch 23390 pretreatment (0.05 - 0.10 mg/kg, iv).

#### Femoral

The thoracic aorta was catheterized with a Millar micro-tip (size 5F) transducer via the right carotid artery for blood pressure monitoring. The right external jugular vein was cannulated with PE-160 tubing for supplemental pentobarbital administration. The left femoral artery was isolated and an electromagnetic flowprobe of the appropriate size (8-11 mm circ.) was placed around the artery and connected to a Caroline Medical electromagnetic flowmeter. Surface ECG leads were placed subcutaneously on the chest and limbs to monitor heart rate. A 25 gauge 3/4" needle bent 90°, 4 mm from the tip, was placed in the femoral artery proximal to the probe and connected to an infusion pump delivering saline at a constant rate of 1 ml/min. Following a saline and bradykinin challenge (0.02 to 0.04 nmoles, in 0.2 ml saline, i.a.), a dose response curve for DPDA was generated by injecting 4-fold increasing doses i.a. ranging from 3 to 48 nmoles in 0.2 ml saline. Test compounds were injected similarly in 4-fold increasing doses up to 3000 nmoles. Compounds were then repeated at their optimal dose following domperidone pretreatment (10-40 µg/kg, iv).

DATA ANALYSIS

Efficacy ratios were determined by division of the optimal response of the test compound by the optimal response of the standard agent. The optimal dose of compound was defined as that which produced a maximum increase in blood flow before causing a change in systemic blood pressure. $ED_{50}$ values were derived by regression analysis from individually generated dose response curves. Potency ratios were the quotients calculated from the division of the standard agent $ED_{50}$ by the $ED_{50}$ of the test compound. This data, together with the information on the percent blockade of optimal doses of the active compounds following antagonist administration, is present in the following Tables. If the inhibition of the test compound was similar to that of dopamine or DPDA, the compound was considered to be a $DA_1$ or $DA_2$ agonist, respectively.

| RENAL $DA_1$ ASSAY | | | | | |
|---|---|---|---|---|---|
| Compound | n | $ED_{50}$ (nmoles) | Relative Potency to $DA_1$ Standard | Relative Efficacy to $DA_1$ Standard | Percent Block Following SCH 23390 |
| Standard: | | | | | |
| Dopamine | 9 | | 1.00 | 1.00 | 100% |
| Test Compound 1 | 3 | 0.87 | 6.44 | 1.10 | 100%(R) |
| Test Compound 2 | 3 | 0.34 | 17.47 | 0.93 | 100%(R) |
| R = reponse was reversed following Schering 23390 blockade. | | | | | |

| RENAL $DA_2$ ASSAY | | | | | |
|---|---|---|---|---|---|
| Compound | n | $ED_{50}$ (nmoles) | Relative Potency to $DA_2$ Standard | Relative Efficacy to $DA_2$ Standard | Percent Block Following Domperidone |
| Standard: | | | | | |
| DPDA | 19 | | 1.00 | 1.00 | 100% |
| test compd 1 | 3 | 0.02 | 327.50 | 1.29 | 0%(n = 2) |
| test compd 2 | 3 | 0.04 | 216.00 | 1.26 | 24%(n = 3) |

The two compounds tested in these assays were:
test compound 1 :
(R)-2-(3,4-dihydroxybenzyl)-1-[6-(N-(4-hydroxyphenethyl)-N-propylamino)hexyl]pyrrolidine dihydrobromide
test compound 2 :
(R)-2-(3,4-dihydroxybenzyl)-1-[6-(N-(3-hydroxyphenethyl)-N-propylamino)hexyl]pyrrolidine dihydrobromide

## EXAMPLE 16

Spontaneous Hypertensive Rat-Diuretic Activity

The compounds of this invention were examined for diuretic activity for 6 hours following oral compound administration in the saline-loaded spontaneously hypertensive rat (SHR). Methods for this assay have been previously described (Rosenkranz, et al, Proc. West. Pharmacol. Soc. 28 :87, 1985). The diuretic and natriuretic effects elicited by these compounds were characterized by an immediate onset of action. Over the course of the 6 hour study, the compounds were found to be as efficacious as the standard diuretic agent, hydrochlorothiazide.

Male spontaneously hypertensive SHR/NCrlBR rats weighing 320-430 gms were divided into four

groups of seven animals. All animals were food- and water-deprived overnight. The following morning, each group of rats was hydrated with deionized water (20 ml/kg, po) forty-five minutes prior to the administration of vehicle or the test compounds.

Following vehicle or drug, the rats were placed in individual metabolic units. Fifteen minutes post-dose the animals were saline-loaded (30 ml/kg, po). Urine was collected at 1, 3 and 6 hour intervals post-saline-load. Urine volumes were measured and sodium and potassium levels were determined by flame photometry.

A two-way analysis of variance with time, treatment and their interaction was run as a repeated measures analysis. A secondary model of one-way analysis of variance (by time) was run using specified contrasts. The p-values for the contrasts were adjusted using Fisher's LSD strategy at each time point.

At one and three hours post oral administration of the test compounds at 10 mg/kg, significant diuresis and natriuresis were observed. Diuresis and natriuresis ($p<0.05$) were observed at the 30 mg/kg dose level at 1, 3 and 6 hours post-dose. Administration of 30 mg/kg, po of test compound 1, produced significant kaliuresis at the 1, 3, and 6 hour post-dose time points.

| TEST COMPOUND 1 | | | | |
|---|---|---|---|---|
| | Urine Volume (ml) n = 7 $\bar{X}$ ± S.D. | | | |
| Cumulative Time (hr) | Control Vehicle[b] | Test Compound, po, mg/kg | | |
| | | 3 | 10 | 30 |
| 1 | 6.1±2.2 | 4.6±1.9* | 9.9±3.6* | 14.7±2.3* |
| 3 | 8.7±1.9 | 7.8±1.4 | 11.4±3.9 | 18.6±2.8* |
| 6 | 10.0±1.9 | 9.4±1.8 | 11.8±3.9 | 19.3±2.7* |
| | Urine $Na^+$ (mEq/sample) n = 7 $\bar{X}$ ± S.D. | | | |
| Cumulative Time (hr) | Control Vehicle[b] | Test Compound, po, mg/kg | | |
| | | 3 | 10 | 30 |
| 1 | 0.03±0.03 | 0.02±0.02* | 0.28±0.13* | 0.61±0.16* |
| 3 | 0.13±0.08 | 0.07±0.03* | 0.51±0.22 | 1.13±0.25* |
| 6 | 0.26±0.15 | 0.25±0.10 | 0.51±0.27 | 1.31±0.27* |
| | Urine $K^+$ (mEq/sample) n = 7 $\bar{X}$ ± S.D. | | | |
| Cumulative Time (hr) | Control Vehicle[b] | Test Compound, po, mg/kg | | |
| | | 3 | 10 | 30 |
| 1 | 0.04±0.02 | 0.03±0.04 | 0.06±0.03 | 0.07±0.02* |
| 3 | 0.10±0.03 | 0.11±0.05 | 0.16±0.08 | 0.24±0.05* |
| 6 | 0.18±0.03 | 0.20±0.07 | 0.22±0.11 | 0.31±0.06 |

*$p<0.05$ as compared to control (SAS statistical analysis program).
[b]2% ethanol and 0.5% tween in deionized water.

| Test Compound 2 | | | | |
|---|---|---|---|---|
| | Urine Volume (ml) n = 7 $\overline{X}$ ± S.D. | | | |
| Cumulative Time (hr) | Control Vehicle[b] | Test Compound, po, mg/kg | | |
| | | 3 | 10 | 30 |
| 1<br>3<br>6 | 5.9±1.8<br>8.8±1.6<br>9.8±1.5 | 5.2±1.4*<br>7.9±0.7<br>9.1±0.8 | 8.9±1.6*<br>11.2±2.0<br>11.7±2.4 | 13.0±2.2*<br>15.7±2.1*<br>15.9±2.1* |
| | Urine $Na^+$ (mEq/sample) n = 7 $\overline{X}$ ± S.D. | | | |
| Cumulative Time (hr) | Control Vehicle[b] | Test Compound, po, mg/kg | | |
| | | 3 | 10 | 30 |
| 1<br>3<br>6 | 0.02±0.02<br>0.13±0.09<br>0.27±0.17 | 0.00±0.00*<br>0.06±0.04*<br>0.19±0.07 | 0.09±0.05*<br>0.26±0.12<br>0.31±0.17 | 0.16±0.05*<br>0.40±0.12*<br>1.45±0.12* |
| | Urine $K^+$ (mEq/sample) n = 7 $\overline{X}$ ± S.D. | | | |
| Cumulative Time (hr) | Control Vehicle[b] | Test Compound, po, mg/kg | | |
| | | 3 | 10 | 30 |
| 1<br>3<br>6 | 0.03±0.01<br>0.11±0.05<br>0.18±0.05 | 0.02±0.01*<br>0.09±0.04*<br>0.17±0.05 | 0.02±0.01*<br>0.10±0.06<br>0.13±0.09 | 0.03±0.02*<br>0.13±0.07*<br>0.17±0.09* |

*p 0.05 as compared to control (SAS statistical analysis program).
[b] 2% ethanol in deionized water.

## EXAMPLE 17

Spontaneous Hypertensive Rats - Antihypertensive Activity

The compounds of this invention were screened for oral antihypertensive activity in the conscious restrained spontaneously hypertensive rat.

Male spontaneously hypertensive rats, SHR/NCrlBr (320-410 g) were fasted overnight. The following morning the left femoral artery and vein of each rat was cannulated under light ether anesthesia. Following surgery, the rats were placed on a rat restraining board and allowed to recover from the effects of the ether for 1 hour. ECG leads were placed on the animal's chest to monitor heart rate. Animals were divided into groups of 4 and dosed with the test compounds.

Blood pressure and heart rate readings were taken at 5 minute intervals for the first 15 minutes and every minutes thereafter for a duration of 4 hour post-compound administration. Data was evaluated by two-tailed paired t-tests.

| TEST COMPOUND 1 | | | |
|---|---|---|---|
| | po, mg/kg | | |
| | 3 | 10 | 30 |
| XΔ ± SD | n = 4 | n = 4 | n = 4 |
| Mean Blood Pressure (mm Hg) | | | |
| Control | 144 ± 4 | 149 ± 6 | 152 ± 5 |
| 1 hour | -17 ± 8* | -17 ± 10* | -20 ± 3* |
| 2 hours | -14 ± 7* | -23 ± 7* | -17 ± 3* |
| 3 hours | -25 ± 17 | -17 ± 18 | -24 ± 9* |
| 4 hours | -30 ± 5* | 26 ± 8* | -26 ± 4* |
| Heart Rate (beats/ min) | | | |
| Control | 415 ± 13 | 428 ± 10 | 431 ± 20 |
| 1 hour | 3 ± 13 | -11 ± 13 | -5 ± 11 |
| 2 hours | 1 ± 22 | -6 ± 11 | 1 ± 17 |
| 3 hours | 0 ± 18 | -6 ± 18 | 0 ± 15 |
| 4 hours | 8 ± 23 | 10 ± 9 | -8 ± 26 |
| Systolic Blood Pressure (mm Hg) | | | |
| Control | 192 ± 10 | 207 ± 10 | 202 ± 11 |
| 1 hour | -29 ± 12* | -29 ± 15* | -23 ± 9* |
| 2 hours | -25 ± 14* | -41 ± 15* | -22 ± 7* |
| 3 hours | -31 ± 16* | -25 ± 27 | -27 ± 9* |
| 4 hours | -38 ± 18* | -44 ± 12* | -37 ± 6* |
| Diastolic Blood Pressure (mm Hg) | | | |
| Control | 115 ± 6 | 119 ± 7 | 123 ± 6 |
| 1 hour | -11 ± 3* | -14 ± 9 | -14 ± 3* |
| 2 hours | -5 ± 8 | -14 ± 8* | -15 ± 5* |
| 3 hours | -13 ± 13 | -13 ± 11 | -16 ± 8* |
| 4 hours | -19 ± 5* | -17 ± 3* | -24 ± 5* |

* = P 0/05 as compared to control

| TEST COMPOUND 2 | | | |
|---|---|---|---|
| | 3 | 10 | 30 |
| XΔ ± SD | n = 4 | n = 4 | n = 4 |
| Mean Blood Pressure (mm Hg) | | | |
| Control | 148 ± 8 | 156 ± 13 | 148 ± 6 |
| 1 hour | -12 ± 18 | -21 ± 3* | -5 ± 9 |
| 2 hours | -7 ± 8 | -21 ± 8* | -14 ± 14 |
| 3 hours | -8 ± 14 | -27 ± 4* | -17 ± 11 |
| 4 hours | 14 ± 9 | -22 ± 12* | 25 ± 21 |
| Heart Rate (beats/ min) | | | |
| Control | 423 ± 18 | 406 ± 15 | 406 ± 15 |
| 1 hour | -5 ± 12 | -11 ± 6* | -1 ± 10 |
| 2 hours | 5 ± 11 | -6 ± 8 | 9 ± 10 |
| 3 hours | 6 ± 18 | -5 ± 11 | 18 ± 15 |
| 4 hours | 10 ± 23 | 1 ± 3 | 20 ± 16 |
| Systolic Blood Pressure (mm Hg) | | | |
| Control | 204 ± 18 | 206 ± 23 | 194 ± 9 |
| 1 hour | -13 ± 25 | -26 ± 6* | -4 ± 14 |
| 2 hours | -9 ± 13 | -28 ± 10* | -18 ± 10* |
| 3 hours | -19 ± 22 | -36 ± 23 | -26 ± 17 |
| 4 hours | -29 ± 19 | -31 ± 20 | -32 ± 20* |
| Diastolic Blood Pressure (mm Hg) | | | |
| Control | 117 ± 8 | 132 ± 13 | 125 ± 4 |
| 1 hour | -8 ± 12 | -19 ± 3* | -6 ± 5 |
| 2 hours | -6 ± 3* | -11 ± 21 | -17 ± 6* |
| 3 hours | -4 ± 17 | -19 ± 8* | -15 ± 5* |
| 4 hours | -9 ± 7* | -16 ± 7* | -20 ± 9* |

* = P 0/05 as compared to control

Peak decreases for compound 1 were about 20% for both mean blood pressure and systolic blood pressure, whereas peak decreases of 17% were recorded for compound 2. The duration of action lasted up to 4 hours. The heart rate was not significantly altered following administration of test compounds 1 or 2.

EXAMPLE 18

TOXICITY

No toxic effects were observed in the tests reported above with compounds of this invention.

The instant invention is disclosed and described herein in what is considered to be the most practical, and preferred embodiments. It is recognized, however, that departures may be made therefrom which are within the scope of the invention, and that obvious modifications will occur to one skilled in the art upon reading this disclosure.

**Claims**

1. A compound of formula (I)

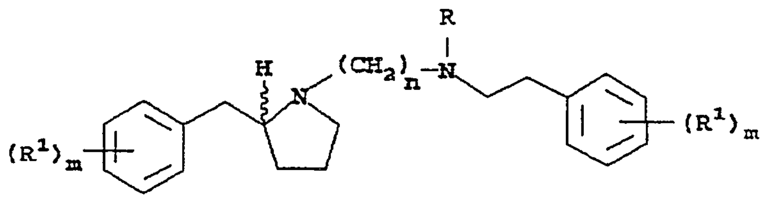

(I)

wherein:
R is hydrogen or lower alkyl;
each $R^1$ is independently hydrogen,

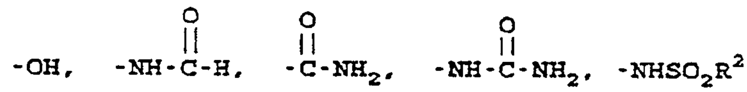

or halo;
$R^2$ is lower alkyl or optionally substituted phenyl; m is an integer of 0, 1, 2, 3, 4, 5, or 6, and n is an integer of from 1 to 10, or pharmaceutically acceptable salts, or (*R*) or (*S*) stereoisomers, or racemic or non-racemic mixtures thereof.

2. A compound of Claim 1 wherein R is lower alkyl, $R^1$ is independently hydrogen or hydroxy in the 3 and/or 4 positions of each phenyl group, m is 3 or 4, and n is 5 or 6.

3. A compound of Claim 2 wherein R is propyl, $R^2$ is hydroxy in the 3 and/or 4 position of each phenyl group, m is 3 or 4, and n is 5 or 6.

4. The compound of Claim 3 wherein m is 4 and n is 5, namely (R)-2-(3,4-dihydroxybenzyl)-1-[5-(N-(3,4-dihydroxyphenethyl)-N-propylamino)pyrrolidine or a pharmaceutically acceptable acid addition salt thereof.

5. The compound of Claim 3, wherein m is 4 and n is 6, namely (R)-2-(3,4-dihydroxybenzyl)-1-[6-(N-(3,4-dihydroxyphenethyl)-N-propylamino)hexyl]pyrrolidine, or a pharmaceutically acceptable acid addition salt thereof.

6. The compound of Claim 3 wherein m is 3 and n is 5, namely (R)-2-(3,4-dihydroxybenzyl)-1-[5-(N-(4-hydroxyphenethyl)-N-propylamino)pentyl]pyrrolidine or a pharmaceutically acceptable acid addition salt thereof.

7. The compound of Claim 3 wherein m is 3 and n is 6, namely (R)-2-(3,4-dihydroxybenzyl)-1-[6-(N-(4-hydroxyphenethyl)-N-propylamino)hexyl]pyrrolidine or a pharmaceutically acceptable acid addition salt thereof.

8. The compound of Claim 3 wherein m is 3 and n is 5, namely (R)-2-(3,4-dihydroxybenzyl)-1-[5-(N-(3-hydroxyphenethyl)-N-propylamino)pentyl]pyrrolidine or a pharmaceutically acceptable acid addition salt thereof.

9. The compound of Claim 3 wherein m is 3 and n is 6, namely (R)-2-(3,4-dihydroxybenzyl)-1-[6-(N-(3-hydroxyphenethyl)-N-propylamino)hexyl]pyrrolidine or a pharmaceutically acceptable acid addition salt thereof.

10. A composition, comprising :
a pharmaceutically acceptable carrier; and
a compound of formula (I):

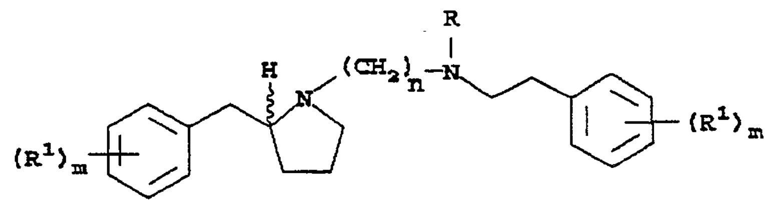

(I)

wherein:
R is hydrogen or lower alkyl;
each $R^1$, is independently hydrogen,

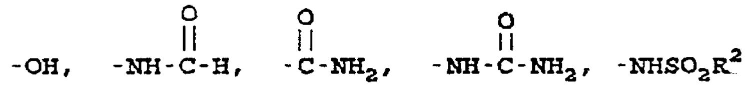

or halo;
$R^1$ is lower alkyl or optionally substituted phenyl; m is an integer of 0, 1, 2, 3, 4, 5, or 6; and n is an integer of from 1 to 10; or a pharmaceutically acceptable salt, (R) or (*S*) stereoisomers, or racemic or non-racemic mixtures thereof.

11. The composition of Claim 10, wherein the composition comprises a pharmaceutically acceptable salt of the compound of formula I wherein R is propyl, $R^1$ is hydroxy in the 3 and/or 4 position of each phenyl group, m is 3 or 4, and n is 5 or 6.

12. The composition of Claim 10, wherein the pharmaceutically acceptable salt of the compound of formula I is the (R) stereoisomer.

13. The composition of Claim 10 wherein the pharmaceutically acceptable salt of the compound of formula I is a salt of (R)-2-(3,4-dihydroxybenzyl)-1-[6-(N-(4-hydroxyphenethyl)-N-propylamino)hexyl]pyrrolidine.

14. A process for the preparation of compounds of formula I:

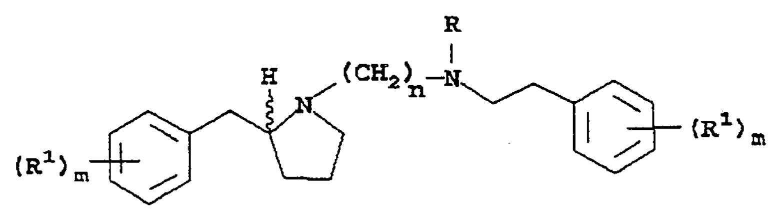

(I)

wherein:
R is hydrogen or lower alkyl;
each $R^1$ is independently hydrogen,

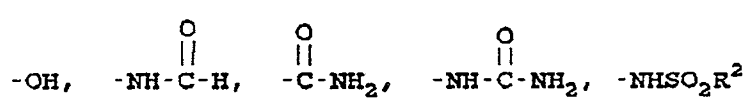

or halo;
$R^2$ is lower alkyl or optionally substituted phenyl; m is an integer of 0, 1, 2, 3, 4, 5, or 6; n is an integer of from 1 to 10; or pharmaceutically acceptable salts, (R) or (S) stereoisomers or racemic or non-racemic mixtures thereof;
which comprises

a) reacting a compound of formula 14

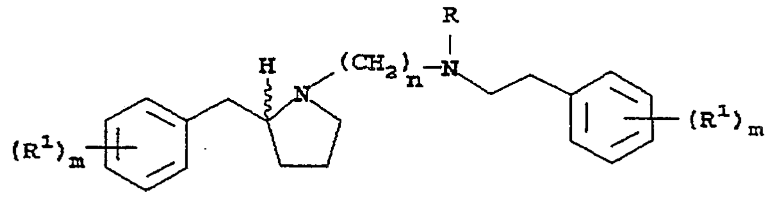

(I)

wherein $R^1$ is OR, R, m, and n are as defined above with a deprotecting agent; or
b) converting a compound of formula I to a pharmaceutically acceptable salt of formula I; or
c) converting a pharmaceutically acceptable salt of formula I to a free compound of formula I; or
d) converting a pharmaceutically acceptable salt of formula I to another pharmaceutically accpetable salt of formula I; or
e) resolving a compound of formula I into its (R) and (S) stereoisomers thereof.

15. The use of a compound according to any one of claims 1-9 in the manufacture of a medicament for treating cardiovascular disorders.

16. The use of a compund according to any one of claims 1-9 in the manufacture of a medicament for treating hypertension.

17. A compound of the formula

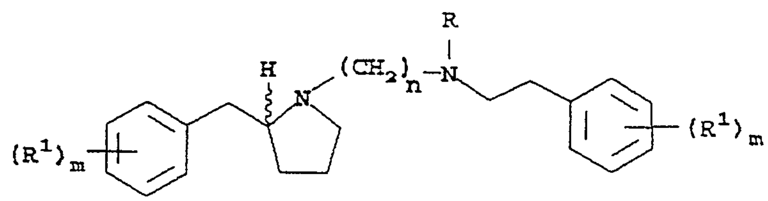

(I)

wherein:
R is hydrogen or lower alkyl;
each $R^1$ is a protected hydroxy group; m is an integer of 0, 1, 2, 3, 4, 5, or 6; and n is an integer of from 1 to 10; or a pharmaceutically acceptable salt, (R) or (*S*) stereoisomers, or racemic or non-racemic mixtures thereof.

18. A process for the preparation of a compound of the formula

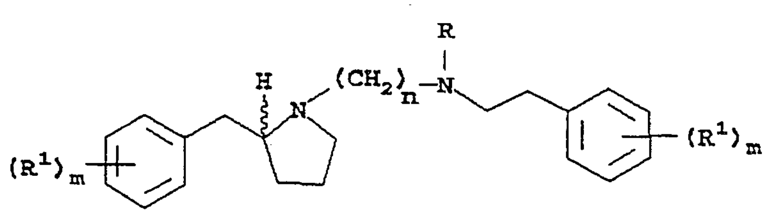

(I)

wherein:
R is hydrogen or lower alkyl;
each $R^1$,is a protected hydroxy group; m is an integer of 0, 1, 2, 3, 4, 5, or 6; and n is an integer of from 1 to 10; or a pharmaceutically acceptable salt, (R) or (*S*) stereoisomers, or racemic or non-racemic mixtures

thereof
which comprises reacting a compound of the formula

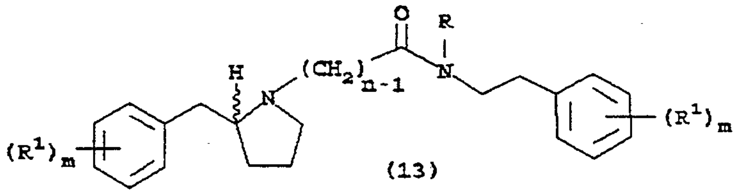

(13)

wherein $R^1$, m, and n are as described above, with a reducing agent.

European
Patent Office

# EUROPEAN SEARCH REPORT

Application Number

**EP 90 12 0713**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | EP-A-0 071 399 (SYNTEX)<br>* Abstract; claim 1 *<br>– – – | 1 | C 07 D<br>207/08<br>A 61 K 31/40 |
| A | GB-A-1 186 660 (HASELTINE)<br>* Page 3, line 95 - page 4, line 5; claim 1 *<br>– – – – – | 1 | |

TECHNICAL FIELDS SEARCHED (Int. Cl.5)

C 07 D 207/00

The present search report has been drawn up for all claims

| Place of search | Date of completion of search | Examiner |
|---|---|---|
| The Hague | 24 January 91 | KISSLER B.E. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document